# EUROPEAN PATENT APPLICATION

(11) **EP 4 035 663 A1**
(43) Date of publication of application: **03.08.2022**
(21) Application number: 21732802.0
(22) Date of filing: 09.03.2021
(51) Int. Cl.: A61K 31/00

(54) **USE OF PROGESTIN IN TREATING CYTOKINE RELEASE SYNDROME**

(30) Priority: 17.12.2020 CN 202011497541
(71) Applicant: Shenzhen Evergreen Therapeutics Co., Ltd., Shenzhen, Guangdong 518052 (CN)
(72) Inventor: HU, Tao, Shenzhen, Guangdong 518052 (CN); DU, Tao Tom, Shenzhen, Guangdong 518052 (CN); DU, Xin, Shenzhen, Guangdong 518052 (CN)
(74) Representative: J A Kemp LLP
(86) International application number: PCT/CN2021/079775
(87) International publication number: WO 2022/126869

(57) **Abstract**

Disclosed is use of hydroxyprogesterone caproate in the manufacture of a medicament for inhibiting cytokine storm, in particular cytokine release from PBMCs induced by anti-CD28 antibody and/or anti-CD3 antibody. The results show that hydroxyprogesterone caproate could inhibit various cytokines release from PBMCs induced by anti-CD28 antibody and/or anti-CD3 antibody in a concentration-dependent manner, and could be a potential drug for the treatment of cytokine storm.

## Description

### TECHNICAL FIELD

The present invention belongs to the field of treatment of immune diseases and infection diseases, and in particular provides use of hydroxyprogesterone caproate in the manufacture of a medicament for inhibiting cytokine storm, especially cytokine release from peripheral blood mononuclear cells (PBMCs).

### BACKGROUD

Cytokine release syndrome (CRS) is a group of clinical syndromes involving activation and dissolving of lymphocytes and release of a large amount of cytokines triggered by infections or therapies with monoclonal antibodies or cytokines. This phenomenon was first discovered in the early 1990s when Muromonab-CD3 monoclonal antibody (OKT3) was used for anti-transplant rejection. In March 2006, in the UK phase I clinical trial of a monoclonal antibody TGN1412, six healthy men in the experimental group experienced multi-organ failure, of which two men developed deep coma, which was later confirmed to be related to the induction of CRS. This incident caused the scientific community and drug regulatory agencies to re-examine the pre-clinical research, especially CRS, of monoclonal antibodies, and put forward corresponding clinical trial guidelines. Early clinical manifestations of CRS are easily confused with infusion reactions. Typical symptoms are usually mild to moderate, and general symptoms include fever, fatigue, myalgia, and arthralgia. Symptoms may relate to skin, respiration, angiocarpy, gastrointestinal tract, coagulation, and nervous systems, etc., such as skin rash, shortness of breath, tachycardia, hypotension, vomiting, diarrhea, bleeding, mental disorders, convulsions or convulsions. Some symptoms are caused due to clearing of antigen-antibody immune complexes by the reticuloendothelial system in the lungs, liver, and spleen. Some patients may have severe reactions or even sudden death. CRS is one of the main causes of death in patients with organ transplantation, novel coronavirus pneumonia, CAR-T and macromolecular drug treatments. It is reported that up to 93% of CAR-T treated patients and 20% of novel coronavirus pneumonia patients would have varying degrees of CRS.

The mechanism for starting CRS has not yet been fully clarified. It is generally believed CRS is caused by the activation of specific inflammatory cells, especially monocytes, macrophages, T cells and B cells. The currently known possible mechanisms of CRS include: 1) binding of soluble antibodies to corresponding antigens on the surface of cells through antigen-specific determinants of the antibodies activates effector cells to release a large amount of cytokines; 2) Fc fragments of antibodies bind to Fc receptors of non-effector cells to produce a similar bystander effect, which activates corresponding binding cells to release cytokines; 3) murine portions of monoclonal antibodies produce "anti-antibody" which activates immunological responses and releases cytokines. Existing clinical observations have shown that the occurrence of CRS in patients has explicit relation with increased levels of at least seven cytokines in serum. The seven cytokines include: interleukin-5 (IL-5), interleukin-6 (IL-6), interleukin-10 (IL-10), interferon-y (IFN-γ), granulocyte-macrophage colony stimulating factor (GM-CSF), fractal chemokine (Fractalkine) and human FMS-related tyrosine kinase 3 ligand (Flt-3L). In addition, since the human immune system is a very complex system, there are cross reactions and activatations of various mechanisms. The occurrence of CRS may be affected by affinity of antibodies, epitope of antibodies, characteristics of antigens, binding of Fc fragments of antibodies to Fc receptors, and even the activation of a series of signal transduction in the intracellular part of the transmembrane protein, so CRS may have various clinical manifestations.

The current clinical treatment of CRS is mainly based on symptomatic treatment. Common treatment measures include: 1) life support: maintaining body temperature and intestinal and external nutrition and energy supply; 2) respiratory support: including general oxygen therapy, mechanical ventilation and surface active substances supply; 3) circulatory support: adjusting the input and output for maintaining the acid-base balance of water-electrolyte, appropriately using colloids, crystals, vasoactive drugs, and heparin for diffuse intravascular coagulation; 4) preventive use of corticosteroids, acetaminophen and antihistamines such as diphenhydramine, etc., or repeated use of them when the disease strikes; 5) Others including hemofiltration and continuous renal replacement therapy, etc. Up to now, there is still a lack of clinically safe and effective drugs direct to CRS pathogenesis. For example, treating CAR-T induced CRS with Tocilizumab, which is an IL-6 receptor antagonist, could increase the incidence and severity of neurotoxicity. When Tocilizumab is not effective for CAR-T patients, glucocorticoids with side effects has to be used for treatment. In view of the above, there is still a great demand for drugs direct to the pathogenesis of CRS in this field.

At present, peripheral blood mononuclear cells (PBMCs) model is the most sensitive CRS model that can be established by stimulating PBMCs in vitro with specific antibodies. Human PBMCs is composed of lymphocytes including T cells, B cells and NK cells, as well as monocytes and a small amount of dendritic cells. Lymphocytes account for about 70-90% of PBMCs, and CD3+ T cells account for the highest proportion, about 45-70%. It is known that lymphocytes are activated and dissolved after the body is stimulated, and release a large amount of cytokines, which is the main pathogenesis of CRS. Therefore, in the present study a human PBMCs stimulation model is used to screen potential drugs that inhibit the release of cytokines.

### SUMMARY OF THE INVENTION

In order to suppress cytokine storms and treat related diseases, as well as for in vitro scientific research, a large number of drug candidates are screened, wherein hydroxyprogesterone caproate in the development stage is a potential drug candidate with high efficacy and low side effects. Test results show that hydroxyprogesterone caproate could inhibit cytokine release from PBMCs induced by various antibodies or stimulating substances in a concentration-dependent manner, so it is a potential drug or agent for treating cytokine storm or inhibiting cytokine release.

In a first aspect, disclosed herein is use of progestogen in the manufacture of a medicament for the treatment of cytokine storm syndrome.

In a second aspect, disclosed herein is a method for inhibiting cytokine storm in a subject in need thereof, comprising administering progestogen to the subject.

In a third aspect, disclosed herein is a pharmaceutical composition for inhibiting cytokine storm syndrome, comprising progestogen.

In some embodiments, the progestogen is hydroxyprogesterone caproate, medroxyprogesterone acetate, and/or progesterone, preferably hydroxyprogesterone caproate.

In some embodiments, the cytokine storm in the method is a cytokine release from PBMCs. The cytokine release may occur in vivo or in vitro.

In some embodiments, the cytokine released from PBMCs comprises one or more selected from IL-2, IL-6, IL-10, TNF-α and IFN-γ.

In some embodiments, the cytokine release syndrome is triggered by an infection, an antibody-based therapy, an organ transplantation or a chimeric antigen receptor T cell (CAR-T) therapy; preferably, the cytokine release syndrome is triggered by an antibody-based therapy, an organ transplantation or a CAR-T therapy.

In some embodiments, the antibody-based therapy comprises use of an anti-CD28 antibody and/or an anti-CD3 antibody; or the CAR-T therapy comprises use of CAR selected from an anti-CD28 antibody and/or an anti-CD3 antibody, or an active fragment thereof.

In some embodiments, the anti-CD28 antibody is ANC28.1/5D10, and the anti-CD3 antibody is OKT3.

As used herein, the term "antibody" comprises various antibodies with respect to a certain antigen in the prior art, including but not limited to monoclonal antibodies, polyclonal antibodies, single-chain antibodies, nanobodies, etc., and the active parts or fragments of these antibodies can be intercepted by those skilled in the art as needed.

As used herein, the term "hydroxyprogesterone caproate" is also known as 17-α hydroxyprogesterone caproate, 17-HPC, or 17-hydroxyprogesterone caproate, and has a CAS number: 630-56-8. The term "hydroxyprogesterone caproate" is abbreviated as HPC in the Embodiments and Drawings.

In some embodiments, the medicament in this application can be in any clinically acceptable dosage form, and can be administered in any clinically acceptable dosage form in the therapy. The specific dosage forms include, but are not limited to, tablets, capsules, oral liquids, injections, powder injections and the like.

In some embodiments, the medicament prepared by the present application may also comprise other known and unknown drugs for treating related diseases. The therapy method of the present application may also comprise use of other known and unknown drugs for treating related diseases. These drugs include but are not limited to: drugs for treating autoimmune diseases, such as immunosuppressants, peroxisome proliferator-activated receptor agonists, sphingosine-1-phosphate receptor agonists, cyclooxygenase inhibitors, antioxidants, anti-tumor necrosis factor therapy, intravenous immunoglobulin, and other therapies; drugs for treating infections, such as antibiotics, antifungal agents, viral replication inhibitors, viral invasion inhibitors, and drugs known and unknown for treating symptoms such as fever, vomiting, and skin problems in related diseases.

In some embodiments, according to the dosage forms prepared/administered, various pharmaceutically acceptable excipients can be used in the medicament, including but not limited to coating materials, solvents, solubilizers, binders, stabilizers, antioxidants, pH regulators and flavoring agents. Those skilled in the art can make a selection in these excipients according to common knowledge in pharmacy.

### RIEF DESCRIPTION OF THE DRAWINGS

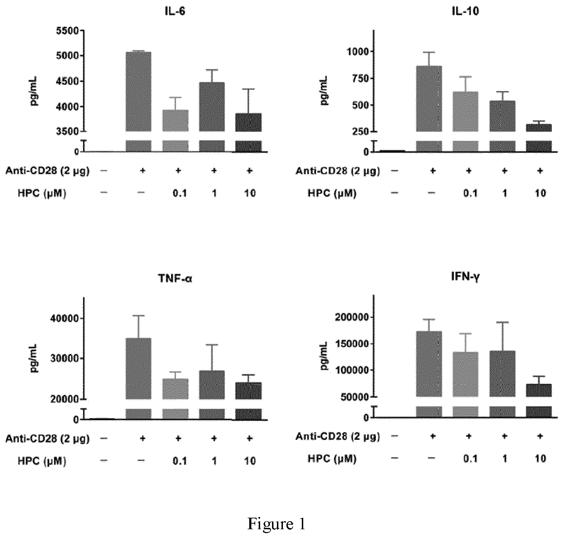

Figure 1 shows inhibition by HPC on IL-6, IL-10, TNF-α and IFN-γ release in human peripheral blood mononuclear cells triggered by anti-CD28 antibody (average values ± standard deviation, n=3);

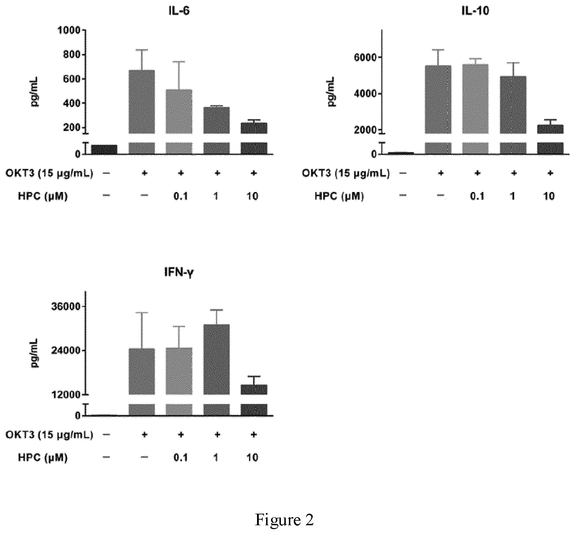

Figure 2 shows inhibition by HPC on IL-6, IL-10 and IFN-γ release in human peripheral blood mononuclear cells triggered by OKT3 (average values ± standard deviation, n=3);

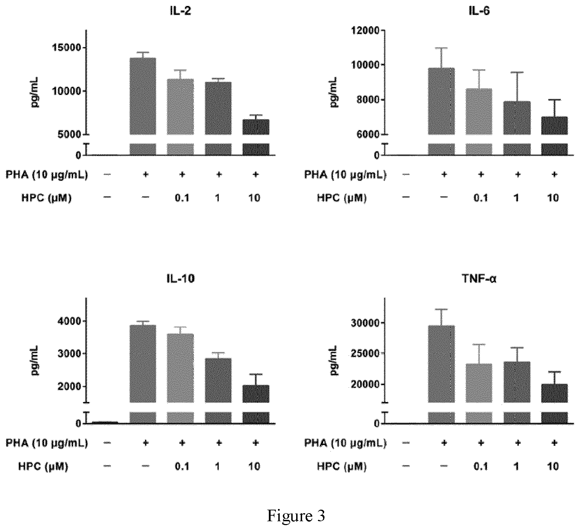

Figure 3 shows inhibition by HPC on IL-2, IL-6, IL-10 and TNF-α release in human peripheral blood mononuclear cells triggered by anti-PHA (average values ± standard deviation, n=3).

### DETAILED DESCRIPTION

The human peripheral blood mononuclear cells (PBMCs) used in the following experiments are derived from Eurofins Discovery (St Charles, MO, USA) and are a mixture of PBMCs from three healthy human subjects (Lot# 98, 99, 101).

### Example 1 Inhibition by hydroxyprogesterone caproate (HPC) on release of cytokines in human peripheral blood mononuclear cells (PBMCs)

### 1) T-cell inflammation inhibition assay protocol using anti-CD28 super agonists First Day

High-binding enzyme-labeled plates are coated with anti-CD28 super agonists (clone ANC28.1/5D10, 2 µg/well) dissolved in PBS 1× and incubated overnight in a biosafety cabinet with lid open and immobilized by air-dry. Cryopreserved PBMCs are drip thawed, pooled and diluted to an appropriate density, and then are inoculated into a U bottom 96-well polypropylene plate (1.2×10⁵ cells per well) with 228 µL per well of culture medium (RPMI 1640, 10% heat inactivated fetal bovine serum, 1% penicillin/streptomycin, 2 mM L-glutamine). The cells are incubated under 5% CO₂ at 37°C for 1 hour before adding HPC. HPC is dissolved in 50% ethanol & PBS, and is further diluted to 20× with cell culture medium. Then HPC is added to the PBMCs in volumes of 12 µL (1×) in triplicate and incubated under 5% CO₂ at 37°C for 16 hours.

### Second Day

After 16 hours of incubation, 200 µL of cells (1×10⁵) treated with HPC or a control reagent are transferred to the anti-CD28 coated enzyme-labeled plates, and incubated under 5% CO₂ at 37°C for 48 hours. For the negative control groups, 200 µLof cells (1×10⁵) are transferred to an enzyme-labeled plate coated with anti-CD28 or isotype IgG1. After 48 hours of incubation, the enzyme-labeled plates are centrifuged at 200×g for 10 minutes. Cell culture supernatants are collected and stored at -80°C until use in analysis.

### 2) T-cell inflammation inhibition assay protocol using anti-CD3 (OKT3) or PHA First Day

Cryopreserved PBMCs are drip thawed, pooled and diluted to an appropriate density, and inoculated into a 96-well polypropylene plate (2×10⁵ cells per well) with 150 µL per well of culture medium (RPMI 1640, 10% heat inactivated fetal bovine serum, 1% penicillin/streptomycin, 2 mM L-glutamine). The cells are incubated under 5% CO₂ at 37°C for 1 hour before adding HPC. HPC is dissolved in 50% ethanol & PBS, and is further diluted to 20× with cell culture medium. Then HPC is added to the PBMCs in volumes of 10 µL (1×) in triplicate and incubated under 5% CO₂ at 37°C for 16 hours.

### Second Day

After incubation for 16 hours, 40 µL of anti-CD3 (OKT3, 3 µg/well or 15 µg/mL) or PHA (10 µg/mL) is added to the wells according to the plate layout. The cells are incubated under 5% CO₂ at 37°C for 48 hours. Thereafter, the plates are centrifuged at 200×g for 10 minutes. Cell culture supernatants are collected and stored at -80°C until use in analysis.

### Cytokines Measured

The cytokine level of each sample is determined using Luminex methodology and carried out in accordance with the manufacturer's protocol. Cytokine levels in the cell culture supernatants are determined in accordance with the manufacturer's protocol using Human Cytokine/Chemokine Magnetic bead panel from Millipore Sigma (catalogue No. HCYTOMAG-60K) with standards range 3.2, 16, 80, 400, 2000, 10000 pg/mL.

The following cytokines are measured: IFN-γ, IL-2, IL-6, IL-10 and TNF-α.

### Experimental Results

1. Anti-CD28 antibody successfully stimulates the release of specific cytokines from human peripheral blood mononuclear cells, but the release of IL-6, IL-10, TNF-α and IFN-γ are significantly inhibited by HPC, and the inhibition effect on IL-10 and IFN-γ release is concentration dependent.
2. OKT3 successfully stimulates the release of specific cytokines from human peripheral blood mononuclear cells, but the release of IL-6, IL-10 and IFN-γ are significantly inhibited by HPC, and the inhibition effect on IL-6 and IL-10 release is concentration dependent.
3. PHA successfully stimulates the release of specific cytokines from human peripheral blood mononuclear cells, but the release of IL-2, IL-6, IL-10 and TNF-α are significantly inhibited by HPC, in a concentration dependent manner.

## Claims

1. Use of progestogen in the manufacture of a medicament for the treatment of cytokine release syndrome.

2. The use according to claim 1, wherein the progestogen is hydroxyprogesterone caproate, medroxyprogesterone acetate, and/or progesterone, preferably hydroxyprogesterone caproate.

3. The use according to claim 1 or 2, wherein the cytokine release syndrome is triggered by an infection, an antibody-based therapy, an organ transplantation or a chimeric antigen receptor T cell (CAR-T) therapy; and preferably, the cytokine release syndrome is triggered by an antibody-based therapy, an organ transplantation or a CAR-T therapy.

4. The use according to claim 3, wherein the antibody-based therapy comprises use of an anti-CD28 antibody and/or an anti-CD3 antibody; or wherein the CAR-T therapy comprises use of CAR selected from an anti-CD28 antibody and/or an anti-CD3 antibody, or an active fragment thereof.

5. The use according to claim 4, wherein the anti-CD28 antibody is ANC28.1/5D10, and the anti-CD3 antibody is OKT3.

6. The use according to any one of claims 1-5, wherein the cytokine is released from peripheral blood mononuclear cells (PBMCs).

7. The use according to claim 6, wherein the cytokine released from PBMCs comprises one or more selected from IL-2, IL-6, IL-10, TNF-α and IFN-γ.

8. A method of inhibiting cytokine release in a subject in need thereof, comprising administering progestogen to the subject.

9. The method according to claim 8, wherein the progestogen is hydroxyprogesterone caproate, medroxyprogesterone acetate, and/or progesterone, preferably, hydroxyprogesterone caproate.

10. The method according to claim 8 or 9, wherein the cytokine release syndrome is triggered by an infection, an antibody-based therapy, an organ transplantation or a chimeric antigen receptor T cell (CAR-T) therapy; and preferably, the cytokine release syndrome is triggered by an antibody-based therapy, an organ transplantation or a CAR-T therapy.

11. The method according to claim 10, wherein the antibody-based therapy comprises use of an anti-CD28 antibody and/or an anti-CD3 antibody; or wherein the CAR-T therapy comprises use of CAR selected from an anti-CD28 antibody and/or an anti-CD3 antibody, or an active fragment thereof.

12. The method according to claim 11, wherein the anti-CD28 antibody is ANC28.1/5D10, and the anti-CD3 antibody is OKT3.

13. The method according to any one of claims 8-11, wherein the cytokine is released from peripheral blood mononuclear cells (PBMCs).

14. The method according to claim 13, wherein the cytokine released from PBMCs comprises one or more selected from IL-2, IL-6, IL-10, TNF-α and IFN-γ.

15. The method according to claim 13 or 14, wherein the cytokine release is triggered by an anti-CD28 antibody and/or an anti-CD3 antibody or an active fragment thereof.

16. A pharmaceutical composition for inhibiting cytokine release syndrome, comprising progestogen.

17. The pharmaceutical composition according to claim 16, wherein the progestogen is hydroxyprogesterone caproate.

18. The pharmaceutical composition according to claim 16 or 17, wherein the cytokine release syndrome is triggered by an infection, an antibody-based therapy, an organ transplantation or a chimeric antigen receptor T cell (CAR-T) therapy; and preferably, the cytokine release syndrome is triggered by an antibody-based therapy, an organ transplantation or a CAR-T therapy.

19. The pharmaceutical composition according to claim 18, wherein the antibody-based therapy comprises use of an anti-CD28 antibody and/or an anti-CD3 antibody; or the CAR-T therapy comprises use of CAR selected from an anti-CD28 antibody and/or an anti-CD3 antibody or an active fragment thereof.

20. The pharmaceutical composition according to claim 19, wherein the anti-CD28 antibody is ANC28.1/5D10, and the anti-CD3 antibody is OKT3.

21. The pharmaceutical composition according to any one of claims 16-20, wherein the cytokine is released from peripheral blood mononuclear cells (PBMCs).

22. The pharmaceutical composition according to claim 21, wherein the cytokine released from PBMCs comprises one or more selected from IL-2, IL-6, IL-10, TNF-α and IFN-γ.
